Europäisches Patentamt

European Patent Office

Office européen des brevets

(11) Publication number: **0 202 409**
**A2**

(12) **EUROPEAN PATENT APPLICATION**

(21) Application number: 86103380.1

(22) Date of filing: 13.03.86

(51) Int. Cl.4: **C12N 11/04** , C12N 1/04 , A21D 8/04 , C12G 1/02 , A23K 1/00

(30) Priority: 25.03.85 US 715959

(43) Date of publication of application:
26.11.86 Bulletin 86/48

(84) Designated Contracting States:
AT BE CH DE FR GB IT LI LU NL SE

(71) Applicant: **MILES LABORATORIES, INC.**
**1127 Myrtle Street**
**Elkhart Indiana 46514(US)**

(72) Inventor: **Enders, George Leonhard, Jr.**
**22797 Bainbridge Drive**
**Elkhart Indiana 46514(US)**
Inventor: **Good, Ivan Carl**
**19908 County Road 18**
**Goshen Indiana 46526(US)**
Inventor: **Kamara, Bassie Jabron**
**234 Village Drive**
**Mishawaka Indiana 46545(US)**
Inventor: **Kim, Hyung Soo**
**25831 Lily Creek Drive**
**Elkhart Indiana 46514(US)**

(74) Representative: **Jesse, Ralf-Rüdiger, Dr. et al**
**Bayer AG Konzernverwaltung RP**
**Patentabteilung**
**D-5090 Leverkusen 1 Bayerwerk(DE)**

(54) **A process for the production of viable and stable dry microorganisms for food and agricultural purposes.**

(57) Disclosed is a method for the microencapsulation of microorganism cultures. The method involves mixing the culture with a bulking agent and water to form a homogeneous wet granulation. The wet granulation is extruded through a die onto the spinning plate of a spheronizing device which results in the formation of discrete spherical particles. These particles can then be coated with a material which provides the microorganism with specific protection while allowing it to carry out its specific function.

EP 0 202 409 A2

# A PROCESS FOR THE PRODUCTION OF VIABLE AND STABLE DRY MICROORGANISMS FOR FOOD AND AGRICULTURAL PURPOSES

## BACKGROUND OF THE INVENTION

Olson, et al disclose in U.S. Patent 4,310,554 a process for accelerating the aging process of cheese by the use of a microencapsulated enzyme or enzyme producing microorganism. The biocatalyst is microencapsulated by means of multiple phase emulsion techniques.

In U.S. Patent 4,242,219, Bogerman, et al disclose a process for preparing enzyme containing particles which involves homogeneously mixing an enzyme in dry from with a hydrophilic organic cohesive material compatible with the enzyme and a building agent which is water soluble carbohydrate of 5 to 12 carbon atoms together with a moisture regulating agent; dividing the resulting mixture into particles and coating the particles with a water repellent agent such as paraffin oil.

In U.S. Patent 3,775,331 there is disclosed a process for the preparation of enzyme spheres. The process involves mixing a normally solid synethetic enzyme with a normally solid synthetic organic surface active agent at a temperature at which the enzyme is stable, mechanically working the mixture through small openings in a die to produce filaments and mechanically spheronizing these filaments by placing them on the revolving friction plate of a spheronizing device of the type presently under consideration.

There is disclosed in U.S. Patent 3,730,841 to Salvatore, et al a method of treating bound enzymes by encapsulating the enzyme in a coating through which the substrate and its conversion product is permeable such as an alginate or cellulose.

Kondo, et al, in U.S. Patent 3,746,621, disclose a method for making enzyme containing microcapsules which involves forming a dispersion of an enzyme, a binder such as gelating, casein, dextrin, gum arabic or cellulose derivatives, a solvent for the binder and a swellable solid which is insoluble in the binder and spray drying the dispersion at a temperature below that at which the enzyme undergoes denaturation.

In U.S. Patent 4,251,632, Chen, et al describe cell aggregates of enzyme producing bacterial cells which have been treated with the cross-linking reaction product of glutaraldehyde, cyanuric halide and a water soluble cationic polyamine and increasing the strength of the aggregate by adding thereto fines generated during the earlier production of a similar such aggregate.

Co-pending application EP-A-83 102 649.7 filed by Jao, et al on March 17, 1983, claims a method for the production of spherically shaped bacterial cell aggregates which involves introducing a long-chain, polyamine, cationic flocculating agent which is an epihalohydrin/polyamine copolymer and a cross-linking agent for the flocculating agent to an aqueous medium containing the bacterial cells to flocculate them and form a cell/-cross-linked polyamine aggregate which is spheronized by extruding it through a die onto the revolving plate of a spheronizing device. This method is especially suitable for immobilizing cells containing glucose isomerase. Thus, the method involves the formation of spheres of immobilized intracellular enzyme rather than viable cells of the microorganism.

## SUMMARY OF THE INVENTION

The present invention is a method for the production of stable, spherical particles of viable microorganisms which comprises the steps of:

a) mixing a spray-dried, freeze-dried or liquid culture concentrate of the viable microorganism with a bulking agent to form a homogeneous wet granulation having a water content of about 35 to 60% on a w/w basis;

b) extruding the wet granulation through a die of reduced cross-sectional area to produce filaments thereof having a diameter approximately the size of the desired spheres onto the rotating plate of a spheronizing device which comprises a milled friction plate as rotor situated in a cylinder such that the cylinder provides a stationary sidewall for the plate while allowing it freedom to rotate;

c) rotating the plate at a tangential speed sufficient to cause the filaments to be disposed against the cylinder wall and be shaped into discrete spherical particles; and

d) drying the spherical particles to a level of from 5 to 10;pc water on a w/w basis.

Optionally there is included the additional step of coating the spherical particles with a material which provides the microorganism with specific protection while allowing it to carry out its desired function.

## DESCRIPTION OF THE INVENTION

Culture concentrates of harmless lactic acid producing bacteria suitable for use in the present invention include, for example, *Streptococcus lactis, S. cremoris, S. diacetilactis, S.thermophilus, Lactobacillus bulgaricus, L.acidophilus, L. helveticus, L. bifidus, L. casei, L. lactis, L. plantarum, L. delbrueckii, L. thermophilus, L. fermentii, Pediococcus cervisiae* and *P. acidilactici* The term microorganism includes the broad class of harmless, i.e., nonpathogenic, acid producing bacteria. In general, such bacteria possess the ability to ferment simple carbohydrates. These can be provided in spray-dried, freeze-dried or liquid concentrate form by using standard procedures known to those skilled in the art for each method of concentration. In addition to lactic acid producing bacteria, other bacteria such as methanogenic bacteria for production of fuel methane, cellulolytic or amylolytic cultures for animal feed applications or others such as *Eubacterium*species for specific metabolites and yeast cultures suitable for use in the wine and baking industry such as *Saccharomyces cerevisiae, S. carlsbergenesis, S. coreanus* and *Torulopis holmii*can be stabilized by this method.

In practicing this invention, the culture concentrate is mixed with a bulking agent which serves the purpose of acting as a carrier to provide texture to the formulation so that it can be extruded and spheronized in later steps of the process. Typically, the bulking agent is used in a ratio of 1 to 3 parts of bulking agent to 3 parts of the viable microorganism on a weight basis. Suitable bulking agents include food grade cellulose; polysaccharides such as maltrin, lactose and starch; nonfat milk solids; caseinate salts, and rice hulls. The culture concentrate is mixed with the bulking agent and sufficient water to form a homogeneous wet granulation having a water content of about 35 to 65% and preferably 38 to 45% on a weight basis . Optionally, a lubricant can be blended into the formulation to reduce friction at the die during extrtusion. Suitable lubricants include mineral oil, polyols (such as glycerol, polyethylene glycol), polysorbates (such as polyoxyethylene sorbitan monolaurate,

polyoxyethylene sorbitan monostearate,

polyoxyethylene sorbitan monooleate),

carbohydrates (such as lactose, fructose) and edible vegetable oils (such as corn, coconut, olive, peanut, soybean). Typically, the lubricant is added in an amount of up to about 5 weight % of the formulation when used.

After thorough mixing to form a homogeneous wet granulation, the material is extruded through a die having a reduced cross-sectional area onto the rotating plate of a spheronizing device which comprises a milled friction plate as rotor situated in a cylinder such that the cylinder provides a stationary sidewall for the plate while allowing it to rotate freely. The extruded granulation, upon being fed from the extruder onto the spinning milled plate, is disposed against the cylinder wall into an annular or doughnut-like shape with a gradient cross-section. The resultant filaments are initially broken down into short pieces, ideally equal to their diameter, by the friction force of the milled plate and also by the intergranular collision and friction of the moving filaments. The characteristic disposition of the filaments results from the centrifugal force of the spinning of the plate and the tangential force of the friction between the filament and the milled plate surface. A smooth spinning surface does not allow the extrudate to roll but just to slide to the periphery of the plate. The type of spheronizing device contemplated for use in the process of this invention can quickly convert the extruded granulation into small, compact, easily handled spheres. The filaments initially break down into pellets. The accelerating and the decelerating pellets within the mass form a pattern of velocity gradients which result in woven rope-like formations. The extrudate is broken down into pellets which are shaped into spheres with a diameter ideally equal to the diameter of the orifice through which the wet granulation was extruded. The spheronizing machine used in the following examples is marketed by Fujio Padal Co., Ltd., Osaka, Japan under the tradename Marumerizer Q-230. This device has a rotor which is 23 centimeters in diameter. Typically, the plate will be rotated at a speed of 100 to 700 revolutions per minute and preferably at a speed of 350 to 500 RPM to provide a tangential velocity of 4.1 to 5.9 meters per second when using a spheronizing device with a disk having a diameter of 9 inches. - (tangential speed = RPM ÷ 60 X Π X diameter of plate). If the speed of rotation is too great, the plate will simply spin the filaments toward its periphery so rapidly and strongly that they will become compressed against the cylinder wall and create undesirable particles, whereas rotating the plate too slowly will not achieve the desired spheronization since there is not provided sufficient momentum within the spinning filaments for collision and friction as a down-sizing and spheronizing force.

After spheronization, the cell aggregate particles are dried to a moisture level of from about 5 to 10%, preferably 6 to 9%, on a weight basis typically in a fluidized bed dryer. The dyring step is preferably carried out at a temperature below that of the microorganism's optimum growth temperature because temperatures at or above the optimum growth temperature will result in a loss of viability. These spherical particles contain viable cells which will retain their viability over long periods of time. They can be used, for example, in the production of cheeses, yogurts, meat preservation, ensilage additives, animal and food supplements and food fermentation for preservation purposes.

We have found that the viability of the cells can be enhanced by applying a coating material. The dried spherical particles are coated with a material which provides the microorganism culture with specific protection while allowing it to carry out its desired function. For example, coatings can be selected which will improve retention of the culture's viability at elevated temperatures or protect the culture's viability against moisture, oxygen or low/high pH environemts. The selection of the coating material will depend on the intended final use of the spheronized culture. Thus, cultures to be used as a probiotic can be coated with enteric coating materials which can protect them from gastric acids and bile salts. Suitable enteric coating materials include formalin-treated gelatin, shellac, zein, cellulose acetate phthalate (CAP), polyvinyl acetate phthalate (PVAP), hydroxypropyl methylcellulose (HPMC), starch and amylose acetate phthalate and styrene-maleic acid copolymers. Agents appropriate for use in protecting the cultures against elevated temperatures include carboxymethylcellulose, polyethylene glycol, carbohydrates, e.g. lactose, dextrin, maltrin, sucrose, xylose, and alginate salts. Coating of the spheres is typically accomplished by use of a fluidized bed dryer or rotating pan spray coater. The spheres obtained may be dried in pans or a fluidized bed dryer. The Wurster process also may be used. Temperatures below or near the culture's optimum growth temperature are used to obtain the desired moisture level. After drying, the spheres are mechanically down-sized by screening using proper sieves with different mesh sizes to obtain a uniform material. The down-sized material may be coated by conventional methods such as spray coating in pans or a fluidized bed dryer using a Wurster apparatus. The preferred method for coating the spheres is a Wurster process which is a one step encapsulation method particularly well suited for single step coating while applying a uniform coating to each particle. The solid spheres are suspended on an air stream which is designed to induce a smooth cyclic flow of particles past the device's air inlet nozzle which atomizes the coating as it is introduced into the fluidized bed dryer. The coated particles are lifted in the air stream which dries the particles as it lifts them away from the spraying nozzle. At the top of the air spout, the particles settle out of the air stream and begin another cycle past the nozzle. The cycle continues until the desired coating thickness has been achieved. The coating material is applied from its aqueous solution or suspension, depending on its chemical nature and solution characteristics.

The method of practicing the invention is further illustrated by the following examples in which all percentages are by weight unless otherwise specified.

EXAMPLE I

Fifty parts by weight of spray-dried, viable *Lactobacillus plantarum* ATCC 39542 containing 2 to 4% by weight of nonfat milk solids and having a moisture content of 8.8% w/w was mixed to homogeneity with 50 parts by weight of Solka floc BW300 (a powdered food grade cellulose from Berlin-Gorham Group of Hackensack, N.J.). One part by weight of glycerol was mixed with 54 parts by weight of distilled water and this mixture was slowly added by droplets to the homogeneous powder mixture at 22°C and mixed very slowly with a Hobart mechanical mixer. This procedure resulted in the formation of a homogeneous granulation at 37% moisture which was fed into a Wenger XBD extruder modified to use a peripheral screen having orifices of 1.2 mm. The mixture was extruded at 22°C using an extruder screw operating at 66 revolutions per minute to form extruded filaments which were immediately placed on the 22,5 mm (9 inch) diamter friction plate a spheronizing device as previously described to provide spheres having diameters of about 1.2 mm by rotating the plate at 350 rpm.

The spheres were dried to a moisture of 9% w/w in a Uni Glatt fluidized bed dryer from Glatt Air Techniques, Inc. The dried spheres were mechanically sized by screening using appropriate sieves to obtain uniform sized particles. Five hundred grams of the uniform size spheres 0,84 mm (-10 + 20) mesh) were coated with 0.28% w/v sodium alginate in aqueous solution by spray coating in a Glatt Uni Glatt fluidized bed dryer using a Wurster insert to yield a final 0.04% w/w coating of sodium alginate. The coated and uncoated products were stored at 4°C, 22°C, 32°C and 37°C and then sampled at time periods to generate stability data.

For testing purposes, each sample of 0.1 gram was transferred into 9.9 ml sterile distilled peptone (0.1%) water in tubes at room temperature. The dissolved samples were vortexed at high speed to break up particles and several dilutions were made in sterile 0.1% peptone water. Dilutions were plated to bracket 1 $^x$ 10$^{10}$ colony forming units per gram of sample and 0.1 ml portions of the dilutions were plated onto APT (All Purpose Tween medium from Difco) agar plates in duplicate. The plates were incubated anaerobically for 48 hours at 37°C and the colony forming units per gram (CFU/gm) determined by the use of a Quebec automatic colony counter and means reported as set out in Table 1.

Table 1
Stability Results
for Example One

| | 4°C | | 22°C | |
|---|---|---|---|---|
| Days | Uncoated | 0.04% W/W Sodium Alginate | Uncoated | 0.04% W/W Sodium Alginate |
| 1 | $1.9 \times 10^{10}$ | $1.9 \times 10^{10}$ | $1.9 \times 10^{10}$ | $1.9 \times 10^{10}$ |
| 8 | $3.0 \times 10^{10}$ | $1.2 \times 10^{10}$ | $9.1 \times 10^{8}$ | $1.0 \times 10^{10}$ |
| 15 | $5.6 \times 10^{10}$ | $1.6 \times 10^{10}$ | $7.7 \times 10^{7}$ | $5.5 \times 10^{9}$ |
| 51 | $1.4 \times 10^{10}$ | $2.5 \times 10^{10}$ | | |

| | 32°C | | 37°C | |
|---|---|---|---|---|
| Days | Uncoated | 0.04% W/W Sodium Alginate | Uncoated | 0.04% W/W Sodium Alginate |
| 1 | $1.9 \times 10^{10}$ | $1.9 \times 10^{10}$ | $1.9 \times 10^{10}$ | $1.9 \times 10^{10}$ |
| 2 | $2.9 \times 10^{9}$ | $5.9 \times 10^{9}$ | $4.4 \times 10^{7}$ | $4.2 \times 10^{7}$ |
| 8 | $2.9 \times 10^{9}$ | $1.8 \times 10^{8}$ | | |
| 14 | | | $2.3 \times 10^{4}$ | $3.4 \times 10^{4}$ |

From Table 1 it can be determined that at 4°C both coated and uncoated particles show similar stability. However, at 22°C the coating stabilizes the viable cell count 70 fold over the uncoated spheres after 25 days. At 32°C and 37°C the sodium alginate did not protect the culture.

EXAMPLE II

A preparation containing viable cells of *L.plantarum* ATCC 39542 liquid concentrate was prepared as follows:

One part by weight of glycerol was mixed with 20 parts by weight of the liquid concentrate. Twelve parts by weight of Solka floc BW300 was then added to the liquid concentrate/glycerol mixture to provide a homogeneous wet granulation. Spherical particles were obtained by the method described in Example I except that a 0.7 mm screen was used on the extruder.

Five hundred grams of the dried spheres (- 20 + 50 mesh size (2.0 mm, 0.30 mm)) were coated with 0.2% w/w carboxy methyl cellulose to yield a final coating of 0.04% w/w. The coated and uncoated spheres were stored at 4°C, 22°C, 32°C and 37°C to determine stability with the stability results being determined by the method of example I.

Table 2
Stability Results
for Example Two

| | 4°C | | 22°C | |
| | | 0.04% W/W | | 0.04% W/W |
| Days | Uncoated | CMC | Uncoated | CMC |
|---|---|---|---|---|
| 1 | $1.9 \times 10^{10}$ | $1.9 \times 10^{10}$ | $1.9 \times 10^{10}$ | $1.9 \times 10^{10}$ |
| 8 | $3.0 \times 10^{10}$ | $2.1 \times 10^{10}$ | $9.1 \times 10^{6}$ | $1.1 \times 10^{10}$ |
| 15 | $5.6 \times 10^{10}$ | $2.3 \times 10^{10}$ | $7.7 \times 10^{7}$ | $3.2 \times 10^{9}$ |
| 23 | | | $1.3 \times 10^{6}$ | $5.8 \times 10^{7}$ |
| 51 | $1.4 \times 10^{10}$ | $3.4 \times 10^{10}$ | | |

| | 32°C | | 37°C | |
| | | 0.04% W/W | | 0.04% W/W |
| Days | Uncoated | CMC | Uncoated | CMC |
|---|---|---|---|---|
| 1 | $1.9 \times 10^{10}$ | $1.9 \times 10^{10}$ | $1.9 \times 10^{10}$ | $1.9 \times 10^{10}$ |
| 2 | $2.9 \times 10^{9}$ | $1.2 \times 10^{10}$ | $4.4 \times 10^{7}$ | $1.9 \times 10^{8}$ |
| 8 | $3.0 \times 10^{4}$ | $1.5 \times 10^{8}$ | | |
| 14 | $1.3 \times 10^{3}$ | $9.5 \times 10^{4}$ | $2.3 \times 10^{4}$ | $6.5 \times 10^{4}$ |

The data presented in Table 2 show that at 4°C both coated and uncoated spheres show similar results. AT 22°C, 32°C and 37°C the carboxymethyl cellulose coating protects the culture to a greater extent.

EXAMPLE III

Twenty parts by weight of viable *Pediococcus acidilactici* liquid concentrate containing 75% w/w moisture was mixed with 1 part by weight of glycerol and 12 parts by weight of Solka floc BW300 to prepare viable dry spherical particles (7%-8% w/w final moisture content) of the microorganism by the method described in Example I. Five hundred grams of the particles obtained at 20 mesh (0.84 mm) size after drying were coated with polyethylene glycol 3000 (50% w/v) in water, giving a 10% w/w coating. The coated and uncoated spheres were kept at 4°C, 22°C and 32°C to determine their stability. Stability results were determined by the procedure described in Example I.

Table 3
Stability Results
for Example 3

| | 4°C | | 22°C | | 32°C | |
| | | 10% W/W | | 10% W/W | | 10% W/W |
| Days | Uncoated | PEG | Uncoated | PEG | Uncoated | PEG |
|---|---|---|---|---|---|---|
| 0 | $2.0 \times 10^{11}$ | $2.0 \times 10^{11}$ | $2.0 \times 10^{11}$ | $2.0 \times 10^{11}$ | $2.0 \times 10^{11}$ | $2.0 \times 10^{11}$ |
| 7 | $2.5 \times 10^{11}$ | $1.9 \times 10^{11}$ | $2.6 \times 10^{11}$ | $1.9 \times 10^{11}$ | $2.6 \times 10^{11}$ | $1.9 \times 10^{11}$ |
| 15 | $1.9 \times 10^{11}$ | $2.5 \times 10^{11}$ | $3.7 \times 10^{11}$ | $4.2 \times 10^{11}$ | $1.3 \times 10^{11}$ | $4.4 \times 10^{11}$ |
| 35 | $2.6 \times 10^{11}$ | $2.0 \times 10^{11}$ | $4.9 \times 10^{11}$ | $3.9 \times 10^{11}$ | $1.0 \times 10^{3}$ | $2.2 \times 10^{10}$ |

The culture used in this example is more hardy than those used in previous examples. This can be seen in the data of Table 3 where at 4°C and 22°C the results are similar whether coated or uncoated. However, after 35 days at 32°C the PEG coated particles did show greater viability.

EXAMPLE IV

The following composition was used to produce dry spherical particles of *Streptococcus cremoris* using the method of Example I: 24 parts by weight of liquid viable concentrate were mixed with 1 part by weight of glycerol and 14 parts by weight of Solka floc BW300. A 0.7 mm screen was used to obtain much smaller particles than in Example I. The spheronized product was dried in the Glatt fluidized bed dryer at an inlet temperature of 28°C and an outlet temperature of 22°C to a moisture level of 8% w/w. The particles were not coated but stored at 4°C and 22°C. Stability results for these particles are set out in Table 4.

Table 4
Stability Results
for Example 4

| Days | 4°C | 22°C |
| --- | --- | --- |
| | Uncoated | Uncoated |
| 0 | $3.0 \times 10^9$ | $3.0 \times 10^9$ |
| 10 | $3.0 \times 10^9$ | $3.0 \times 10^9$ |
| 42 | $5.6 \times 10^9$ | $1.6 \times 10^8$ |
| 76 | $3.2 \times 10^9$ | $1.0 \times 10^6$ |

The data of Table 4 demonstrate that the bacterial cells in the uncoated particles retain full viability for 76 days at 4°C and partial viability at 22°C for 76 days.

EXAMPLE V

With much attention focussed on the relation of gut microecology to animal and human health, species of lactic acid producing bacteria have been reported to influence the composition or types of microorganisms in the gut. For many of these microorganisms, the stomach is a hostile environment where a number of factors or compounds discourage their survival. These factors include pH, fatty acids as well as other organic and inorganic acids.

The cultures of this invention in the form of spheres can be protected against the gastric environment by coating with a pH sensitive film or enteric coating. Enteric coatings are those which remain undissolved in the stomach but will readily dissolve and release the culture on encountering the small intestine. Their main function is to protect the cultures from inactivation in the stomach. The pH sensitive film or enteric coating must be water dispersible and nontoxic to the microorganism.

In practicing the above embodiment of this invention, 30 grams of polyvinyl acetate phthalate - (PVAP) was added slowly to 200 milliliters of distilled water at room temperature (20° -24°C) with constant mixing over 30 minutes. High shear mixing was needed to ensure proper dispersion. After 30 minutes, 1.2 milliliters of a 30% United States Pharmacoepia grade purity solution of ammonium hydroxide was added with stirring. The resulting suspension was passed through a 40 mesh (0.42 mm) screen before spraying. Uncoated product - (500 grams) for Example I was coated with the 200 milliliters of PVAP solution in the Glatt fluidized bed dryer and dried for 15 minutes at an inlet temperature of 30°C and an outlet temperature of 24°C.

The coated and uncoated *L.plantarum*spheres were exposed for various periods of time to simulated (U.S. Pharmacoepia formulation) intestinal fluids for 30 minutes followed by a quantitative assay for viability the results of which are set out in Table 5.

### Table 5
### Gastric Fluids Effect

| Hrs in Gastric Fluid | PVAP Coated Spheres | Uncoated Spheres |
|---|---|---|
| 0.0 Hr. | $4.6 \times 10^{10}$ | $5 \times 10^{8}$ |
| 0.5 Hr. | $3.8 \times 10^{10}$ | $1.3 \times 10^{8}$ |
| 1.0 Hr. | $4.4 \times 10^{10}$ | |
| 2.0 Hr. | $4.9 \times 10^{10}$ | $9.1 \times 10^{7}$ |
| 4.0 Hr. | $6.0 \times 10^{10}$ | $5.1 \times 10^{4}$ |
| 6.0 Hr. | $4.4 \times 10^{10}$ | $1.0 \times 10^{2}$ |

From Table 5 it can be determined that the PVAP coated particles retained full viability for 6 hours in this in vitro study whereas the uncoated particles showed a dramatic decrease in the count of viable cells.

## Claims

1. A method for the production of spherical particles of viable microorganisms which comprises the steps of:

a) mixing a spray-dried, freeze-dried or liquid culture concentrate of the viable microorganism with a bulking agent and water to form a homogenous wet granulation having a water content of about 35% to 60% on a w/w basis;

b) extruding the wet granulation through a die of reduced cross-sectional area to produce filaments thereof having a diameter approximately the size of the desired spheres and placing them on the rotating plate of a spheronizing device which comprises a milled friction plate as rotor situated in a cylinder such that the cylinder provides a stationary sidewall for the plate while allowing it freedom to rotate;

c) rotating the plate at a tangential velocity sufficient to cause the extruded granulation to be disposed against the cylinder wall and be shaped into discrete spherical particles; and

d) drying the spherical particles to a water level of from 5% to 10% water on a w/w basis.

2. The process of Claim 1 which includes the additional step of coating the spherical particles with a material which provides the microorganism culture with specific protection while allowing it to carry out its desired function.

3. The method of Claims 1 or 2 wherein the micro-organism is a methanogenic bacteria or a cellulolytic or amylolytic bacteria suitable for use in animal feed application or a yeast culture suitable for use in the wine and baking industry from the genera and species Saccharromyces cerevisiae, S. carlsbergenesis, S. coreanus or Torulopis holmii.

4. The method of any of the Claims 1 to 3 wherein the bulking agent is a cellulose product such as rice hulls or powdered food grade cellulose, nonfat milk solids, caseinate salts or polysaccharide such as maltrin, lactose or starch.

5. The method of any of the Claims 1 to 4 wherein the lubricant is added to the culture concentrate when forming the wet granulation.

6. The method of any of the Claims 1 to 5 wherein the spherical particle is coated with a material which protects the culture from gastric acids and bile salts.

7. The method of Claim 6 wherein the coating material is formalin-treated gelatin, shellac, zein, cellulose acetate phthalate (CAP), polyvinyl acetate phthalate (PVAP), hydroxypropyl methylcellulose (HPMC), starch and amylose acetate phthalate and styrene-maleic acid copolymers.

8. Spherical particles containing viable microorganism cells prepared by the method of

a) mixing a spray-dried, freeze-dried or liquid culture concentrate of the viable microorganism with a bulking agent and water to form a homogeneous wet granulation having a water content of about 35% to 60% on a w/w basis;

b) extruding the wet granulation through a die of reduced cross-sectional area to produce filaments thereof having a diameter approximately the size of the desired spheres and placing them on the rotating plate of a spheronizing device which comprises a milled friction plate as rotor situated in a cylinder such that the cylinder provides a stationary sidewall for the plate while allowing it freedom to rotate;

c) rotating the plate at a tangential velocity sufficient to cause the extruded granulation to be disposed against the cylinder wall and be shaped into discrete spherical particles; and

d) drying the spherical particles to a water level of from 5% to 10% water on w/w basis.

9) a spherical particle containing viable cells of a nonpathogenic microorganism and a bulking agent to 3 parts of the viable microorganism said particle having a uniform surface coating of a material which provides the microorganism with specific protection while allowing it to carry out its desired function.

10. A method for the production of stable, spherical particles containing a culture concentrate of a lactic acid producing bacteria which comprises the steps of:

a) mixing a spray-dried concentrate containing a bulking agent in an amount of 1 to 3 parts by weight bulking agent to 3 parts of the bacteria and water to form a homogeneous wet granulation having a water content of from 38% to 45% on a w/w basis;

b) extruding the wet granulation through a die of reduced cross-sectional area to produce filaments thereof having a diameter approximately the size of the desired spheres and placing them on the rotating plate of a spheronizing device which comprises a milled friction plate as rotor situated in a cylinder such that the cylinder provides a stationary sidewall for the plate while allowing it freedom to rotate;

c) rotating the plate at a tangential speed of 4.1 to 5.9 meters per second to cause the extruded granulation to be disposed against the cylinder wall and be shaped into discrete spherical particles;

d) drying the spherical particles to a water level of from 6% to 9% water on a w/w basis at a temperature below the optimal growth temperature of the bacteria; and

e) coating the sphericals with a material which provides the bacterial culture with specified protection while allowing it to carry out its desired function.